# EUROPEAN PATENT SPECIFICATION

(11) **EP 3 024 551 B1**
(45) Date of publication and mention of the grant of the patent: **13.03.2024**
(21) Application number: 14744317.0
(22) Date of filing: 25.07.2014
(51) Int. Cl.: A61K 8/31, A61Q 5/06, A61K 8/898, C08L 83/08, A61K 8/33, A61K 8/34, C08G 77/18, C08G 77/26

(54) **PROCESS FOR TREATING THE HAIR WITH AT LEAST ONE SILICONE FUNCTIONALIZED WITH AT LEAST ONE ALKOXYSILANE UNIT, A PARTICULAR SOLVENT AND LESS THAN 2% WATER**
VERFAHREN ZUR BEHANDLUNG DER HAARE MIT MINDESTENS EINEM SILIKON, FUNKTIONALISIERT MIT MINDESTENS EINER ALKOXYSILANEINHEIT, EINEM BESTIMMTEN LÖSUNGSMITTEL UND WENIGER ALS 2 % WASSER
PROCÉDÉ DE TRAITEMENT CAPILLAIRE AVEC AU MOINS UN SILICONE FONCTIONNALISÉ AVEC AU MOINS UNE UNITÉ ALCOXYSILANE, UN SOLVANT PARTICULIER ET MOINS DE 2 % D'EAU

(30) Priority: 26.07.2013 FR 1357380
(43) Date of publication of application: 01.06.2016
(73) Proprietor: L'OREAL, 75008 Paris (FR)
(72) Inventor: PLOS, Grégory, F-75018 Paris (FR); BOUCHARA, Anne, F-75013 Paris (FR); LERDA, Patrice, F-92600 Asnières (FR)
(74) Representative: L'Oreal
(86) International application number: PCT/EP2014/066018
(87) International publication number: WO 2015/011259

(56) References cited:
- WO-A2-2013/068967
- WO-A2-2013/068968
- WO-A2-2013/068979

## Description

The invention relates to a process for treating the hair using a particular composition.

Two major categories of hair shaping products are generally used: styling products and perming products.

Styling products allow non-permanent shaping of the hair. They are used on wet or dry hair before shaping by hand or using a brush or a comb. They are in the form of gels, foams, waxes, pastes, lacquers or sprays. After they have been applied to the hair and after drying, these products harden substantially. This is reflected by an unnatural, embodied, dry feel required for the hold and volume of the hairstyle. Moreover, they do not show good resistance to moisture, and a head of hair loses its shape when exposed to a humid atmosphere, all the more so in a hot and humid atmosphere. Thus, firstly, the general shape of the hairstyle is rapidly lost and, secondly, the hair becomes frizzy, more particularly for hair that is naturally frizzy.

To improve the feel, it is known practice to use silicones, or silicone derivatives, in particular amino silicones and amino silicones bearing silanol functions that are capable of reacting together to form new bonds. Silicones give a natural, soft, non-greasy and non-set feel. They may also be partially water-resistant, which makes it possible to conserve the soft, natural feel. However, they cannot afford shaping of the head of hair or combat the appearance of frizziness, unless a very large amount of product is applied, which gives a greasy feel. As they only sparingly compensate for the dry feel when they are combined with other types of styling product such as fixing polymers, the combination of these two techniques remains relatively unsatisfactory.

Moreover, these styling products are removed on shampooing. They therefore need to be applied daily.

Perming products allow long-lasting shaping of a head of hair.

Generally, the technique used for permanently reshaping the hair consists, in a first stage, in opening the -S-S- disulfide bonds of keratin (cystine) by applying to the hair, which has been placed under tension beforehand (with curlers and other tensioning means), a reducing composition (reduction step) and then, preferably after having rinsed the head of hair thus treated, in reconstituting the said disulfide bonds in a second stage by applying to the hair, which is still under tension, an oxidizing composition (oxidation step, also known as the fixing step) so as to finally give the hair the desired shape.

The new shape given to the hair by a chemical treatment such as that above is long-lasting over time and especially withstands the action of washing with water or with shampoos.

However, such a technique is not entirely satisfactory. Specifically, this technique is very effective for modifying the shape of the hair, but is very degrading to hair fibres.

These two systems do not afford sufficient cosmeticity and/or durability of the effect obtained.

Document WO 2011/080 034 describes compositions comprising a macromolecule with an alkoxysilyl function and a trialkoxysilane, for treating the hair, especially for temporarily reshaping keratin fibres, which show good resistance to washing. WO 2013/068968 is directed to a cosmetic or dermatological composition of soil gel up and / or caring for keratin materials. WO2013068979 which is also directed to cosmetic or dermatological composition of sol/gel type for making up and/or caring keratin materials. However, the cosmetic properties given to the hair are not entirely satisfactory.

Consequently, there is still a need for a hair treatment process for giving the hair the desired shape in a long-lasting manner, while at the same time having very good cosmetic properties.

It is an aim of the present invention precisely to satisfy these needs.

According to one of its aspects, the invention relates to a hair treatment process using a composition as defined in claims.

The present invention also relates to the use of the composition as defined previously, for treating the hair, and in particular for shaping the hair.

The composition used in the process of the invention comprises one or more polymers containing ilicone units and bearing alkoxy-(aminomethyl)-silyl functional groups.

The polymer(s) containing silicone units and bearing alkoxy-(aminomethyl)-silyl functional groups are preferably of formula (I) below: 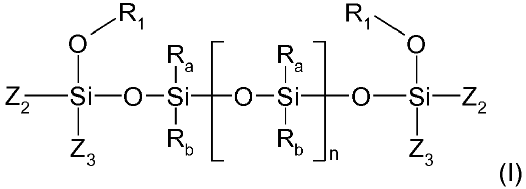 in which:
- Z₂ represents a group CH₂-NR₃R₄;
- Z₃ represents a group OR₅ or R₆;
- R₂ and R₃, which may be identical or different, represent a hydrogen atom or a group R₇;
- R₁, R₅, R₆ and R₇, which may be identical or different, represent a C₁-C₆ alkyl group and R₄ represents a C₁-C₆ alkyl group or a C₅-C₆ cycloalkyl group;

R₃ and R₄ possibly forming, with the nitrogen atom that bears them, a 5- to 8-membered heterocycle comprising from 1 to 3 heteroatoms,
Rₐ and R_{b}, which may be identical or different, represent a C₁-C₂ alkyl group,
n represents an integer greater than 1.

Preferably, the C₁-C₆ alkyl groups are methyl or ethyl groups.

Preferably, R₁ is an ethyl group.

When R₄ represents a C₅-C₆ cycloalkyl group, it preferably represents a C₆ cycloalkyl group such as cyclohexyl.

Preferably, n ranges from 1 to 10 000, more preferably from 5 to 1000 and more preferentially from 8 to 400.

According to a particular embodiment of the invention, Z₂ represents a group -CH₂-NR₃R₄, R₄ represents an alkyl group, preferably a cyclohexyl, R₃ represents a hydrogen atom and R₅ represents an ethyl group.

According to another particular embodiment of the invention, R₃ and R₄ form, with the nitrogen that bears them, a cyclic group, preferably morpholino, and R₅ represents an ethyl group.

Preferably, SiRₐR_{b}-[OSiRₐR_{b}]n- of formula (I) is a unit derived from a linear silicone with a weight-average molecular weight of between 400 and 25 000.

As examples of polymers corresponding to formula (I), mention will be made of:

### - polymers of formula (Ia)

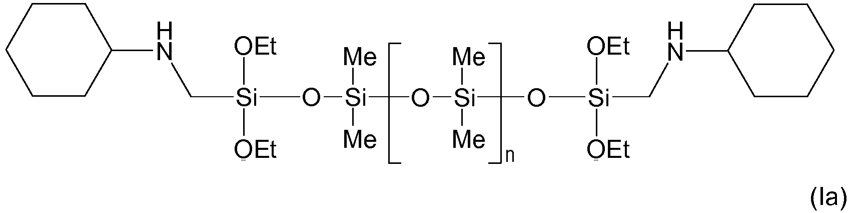

The polymers of formula (Ia) may be obtained by reacting a silicone bearing hydroxyl end groups with triethoxycyclohexylaminomethylsilane especially according to the techniques described in document WO 2005/108 495.

According to a particular example, polymer (laa), corresponding to formula (Ia), is obtained according to the operating scheme presented above, starting with a silicone with a weight-average molecular mass Mw of 4750 g/mol.

### - polymers of formula (Ib)

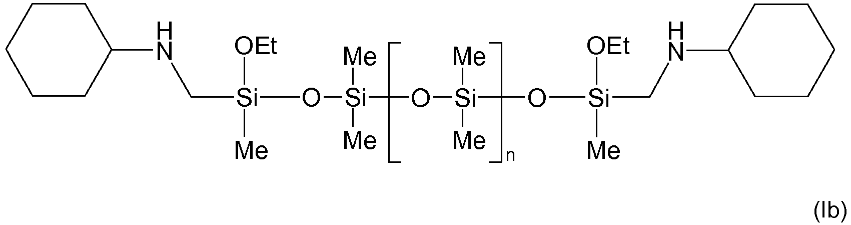

The polymers of formula (Ib) may be obtained by reacting a silicone bearing hydroxyl end groups with diethoxycyclohexylaminomethylmethylsilane especially according to the techniques described in document WO 2005/108 495.

According to a particular example, polymer (Iba), corresponding to formula (Ib), is obtained according to the operating scheme presented above, starting with a silicone with a weight-average molecular mass Mw of 4750 g/mol.

### - polymers of formula (Ic)

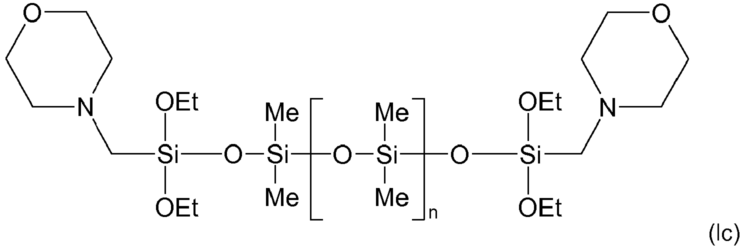

The polymers of formula (Ic) may be obtained by reacting a silicone bearing hydroxyl end groups with triethoxymorpholinomethylsilane especially according to the techniques described in document WO 2009/019 165.

According to a particular example, polymer (Ica), corresponding to formula (Ic), is obtained according to the operating scheme presented above, starting with a silicone with a weight-average molecular mass Mw of 4750 g/mol.

According to another particular example, polymer (Icb), corresponding to formula (Ic), is obtained according to the operating scheme presented above, starting with a silicone with a weight-average molecular mass of 10 600 g/mol.

According to another particular example, polymer (Icc), corresponding to formula (Ic), is obtained according to the operating scheme presented above, starting with a silicone with a weight-average molecular mass of 14 600 g/mol.

According to another particular example, polymer (Icd), corresponding to formula (Ic), is obtained according to the operating scheme presented above, starting with a silicone with a weight-average molecular mass Mw of 21 100 g/mol.

According to another particular example, polymer (ice), corresponding to formula (Ic), is obtained according to the operating scheme presented above, starting with a silicone with a weight-average molecular mass Mw of 550 g/mol.

According to another particular example, polymer (Icf), corresponding to formula (Ic), is obtained according to the operating scheme presented above, starting with a silicone with a weight-average molecular mass Mw of 1000 g/mol.

According to another particular example, polymer (Icg), corresponding to formula (Ic), is obtained according to the operating scheme presented above, starting with a silicone with a weight-average molecular mass Mw of 1200 g/mol.

According to another particular example, polymer (Ich), corresponding to formula (Ic), is obtained according to the operating scheme presented above, starting with a silicone with a weight-average molecular mass Mw of 1700 g/mol.

### - polymers of formula (Id)

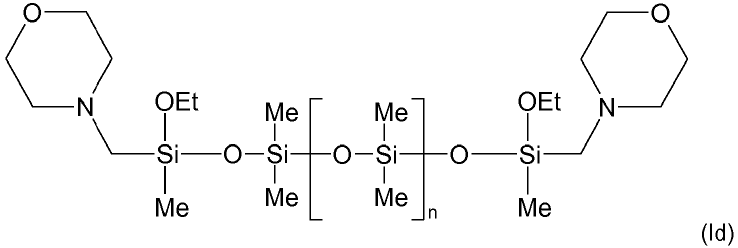

The polymers of formula (Id) may be obtained by reacting a silicone bearing hydroxyl end groups with diethoxymorpholinomethylmethylsilane especially according to the techniques described in document WO 2009/019 165.

According to a particular example, the polymer of formula (Ida), corresponding to formula (Id), is obtained according to the operating scheme presented above, starting with a silicone with a weight-average molecular mass Mw of 4750 g/mol.

According to another particular example, the polymer of formula (Idc), corresponding to formula (Id), is obtained according to the operating scheme presented above, starting with a silicone with a weight-average molecular mass Mw of 10 600g/mol.

According to another particular example, the polymer of formula (Idb), corresponding to formula (Id), is obtained according to the operating scheme presented above, starting with a silicone with a weight-average molecular mass Mw of 14 600 g/mol.

According to a particular example, the polymer of formula (Idd), corresponding to formula (Id), is obtained according to the operating scheme presented above, starting with a silicone with a weight-average molecular mass Mw of 21 100 g/mol.

The content of the polymer(s) containing silicone units and bearing alkoxy-(aminomethyl)-silyl functional groups, preferably of formula (I), in the composition is between 0.5% to 30% by weight and more particularly from 1% to 10% by weight relative to the total weight of the composition.

The process according to the invention may also use one or more catalysts for catalysing the hydrolysis-condensation reactions of the alkoxysilane functions of the polymer containing silicone units and bearing alkoxy-(aminomethyl)-silyl functional groups.

The catalyst may be chosen from acids and bases.

The acid may be chosen from mineral acids and organic acids.

The acid may be chosen in particular from lactic acid, acetic acid, citric acid, tartaric acid, hydrochloric acid, sulfuric acid and phosphoric acid, preferably hydrochloric acid.

The base may be chosen from mineral bases and organic bases.

The base may be chosen from ammonia and sodium hydroxide.

The catalyst may also be chosen from alkoxysilane monomers, optionally bearing an amine function, such as aminopropyltriethoxysilane or octyltriethoxysilane.

The catalyst may be present in the composition used in the process according to the invention, or it may be mixed at the time of use with the composition, or alternatively may be applied sequentially to the hair before or after the composition.

The catalyst(s) may represent from 0.0001% to 10% by weight, preferably from 0.001% to 5% by weight and more particularly from 0.01% to 2% by weight relative to the total weight of the composition containing them.

As indicated previously, the composition used in the process according to the invention comprises less than 5% water. The composition may be aqueous or anhydrous. If the composition is aqueous, it thus comprises less than 5% water, better still less than 3% water and even better still less than 2% water by weight relative to the total weight of the composition.

The composition used in the process according to the invention comprises one or more organic solvents.

The organic solvent(s) are preferably chosen from alcohols, alkanes, esters and silicones, and mixtures thereof.

The alcohols are linear or branched C₁-C₆ monoalcohols or polyols.

The esters may be natural or synthetic.

The esters may be chosen especially from plant oils and esters of fatty acids or of fatty alcohols, such as isopropyl myristate.

The alkanes may be chosen especially from linear or branched C₆-C₁₅ alkanes and liquid paraffins.

The silicones may be chosen especially from cyclic silicones comprising from 4 to 6 silicon atoms and linear polydimethylsiloxanes.

Preferably, the organic solvent is chosen from ethanol, propanol, isopropanol, glycerol, undecane, tridecane, isododecane, isopropyl myristate, ethyl adipate, ethyl acetate, linear low-molecular-weight silicones or cyclic silicones such as cyclopentasiloxane, and also mixtures thereof. According to a preferred embodiment, the solvent is chosen from ethanol, isopropanol and glycerol.

The organic solvents used in the process of the invention are liquids which preferably have a viscosity at 25°C and at atmospheric pressure of less than or equal to 100 cSt.

The organic solvent(s) may represent from 10% to 99.8%, preferably from 30% to 98% by weight and better still from 35% to 95% by weight relative to the total weight of the composition.

The composition may be in the form of a solution, a dispersion or an emulsion. The polymer may be emulsified as an oil-in-water or water-in-oil emulsion or as a multiple emulsion.

The composition may also contain one or more additives chosen from conditioning agents, nonionic, anionic and amphoteric surfactants, vitamins and provitamins including panthenol, water-soluble and liposoluble sunscreens, fillers and solid particles, for instance mineral and organic, coloured or uncoloured pigments, nacreous agents and opacifiers, glitter flakes, mineral fillers, dyes, sequestrants, plasticizers, solubilizers, acidifying agents, basifying agents, mineral and organic thickeners, antioxidants, antifoams, moisturizers, emollients, hydroxy acids, penetrants, fragrances and preserving agents.

Needless to say, a person skilled in the art will take care to select the optional additional compounds and/or the amount thereof such that the advantageous properties of the compositions used according to the invention are not, or are not substantially, adversely affected by the envisaged addition.

The composition may be in the form of a foam, a gel, a serum, a cream, a paste, a wax, a liquid lotion or a lacquer.

The composition may be packaged in a pump-dispenser bottle or in an aerosol device.

When it is packaged in an aerosol-type device, the liquid phase/propellant weight ratio of the pressurized composition of the present invention is preferably between 50 and 0.05, and in particular between 50 and 1.

For the aerosol formulations, any liquefiable gas that is usually used in aerosol devices will be used as propellant gas. Use will be made especially of dimethyl ether, C₃-C₅ alkanes, chlorinated and/or fluorinated, halogenated or non-halogenated, and volatile, hydrocarbons usually used in aerosol devices. Carbon dioxide, nitrous oxide, nitrogen or compressed air, or mixtures thereof, may also be used as propellant.

Preferably, the compound(s) constituting the propellant gas used are chosen from non-halogenated C₃-C₅ alkanes, such as propane, n-butane and isobutane, halogenated, and in particular chlorinated and/or fluorinated, C₃-C₅ alkanes, such as 1,1-difluoroethane, and mixtures thereof.

According to a particularly preferred embodiment, the alkane(s) of the propellant gas are non-halogenated. Even more preferentially, the propellant gas is dimethyl ether or a mixture of propane, n-butane and isobutane.

In the case of aerosol foams, the composition introduced into the aerosol device may, for example, be in the form of a lotion, or dispersions or emulsions which, after dispensing from the aerosol device, form foams to be applied to keratin materials.

These foams are preferably sufficiently stable not to rapidly liquefy and preferably must also rapidly disappear, either spontaneously or during the massaging which serves to make the composition penetrate into keratin materials and/or to distribute the composition over keratin materials and more particularly the head of hair and/or the hair.

In the case of aerosol foams, the composition according to the invention may also contain at least one cationic, nonionic, anionic or amphoteric surfactant.

The propellant gas is present in the composition according to the invention in proportions preferably ranging from 1% to 99% by weight, more preferentially from 1.5% to 50% by weight and better still from 2% to 30% by weight relative to the total weight of the composition.

The aerosol device used for packaging the composition may be made up of two compartments, consisting of an outer aerosol can comprising an inner bag hermetically sealed to a valve. The composition is introduced into the inner bag and a compressed gas is introduced between the bag and the can at a pressure sufficient to make the product come out in the form of a spray through a nozzle orifice. Such a device is sold, for example, under the name EP Spray by the company EP-Spray System SA. The said compressed gas is preferably used at a pressure of between 1 and 12 bar and even better still between 9 and 11 bar.

In the process according to the invention, the composition is applied to wet or dry hair.

Depending on the desired type of hairstyle, it is applied in combination with a heating tool or at room temperature.

The heating tool may be a straightening iron, a curling iron, a crimping iron, a waving iron, a hood or a hairdryer.

The composition may or may not, as indicated above, additionally contain a catalyst that may be chosen from acids and bases.

According to a particular embodiment, the application may be performed in a single stage. In this case, a composition containing both one or more polymers containing silicone units and bearing alkoxy-(aminomethyl)-silyl functional groups, preferably of formula (I), one or more organic solvents, and less than 5% by weight of water relative to the total weight of the composition, and optionally one or more catalysts as defined previously, will be applied.

In this one-stage embodiment, the composition applied to the hair may result from the mixing of a composition comprising one or more polymers containing silicone units and bearing alkoxy-(aminomethyl)-silyl functional groups, preferably of formula (I), one or more organic solvents, and less than 5% by weight of water relative to the total weight of the composition, and of a composition comprising one or more catalysts as defined previously.

According to another embodiment, the application may be performed in two stages: in a step (A), the composition comprising one or more catalysts as defined previously is applied, in a step (B), the composition comprising one or more polymers containing silicone units and bearing alkoxy-(aminomethyl)-silyl functional groups, preferably of formula (I), one or more organic solvents chosen from alcohols, alkanes, esters and silicones, and mixtures thereof, and less than 5% by weight of water relative to the total weight of the composition is applied; in this embodiment, step (A) may be performed, followed by step (B), or alternatively step (B) may be performed, followed by step (A), with or without intermediate drying. Preferably, step (A) is performed, followed by step (B). In this particular embodiment, intermediate drying is preferably performed.

The process according to the invention may be performed using one or more compositions packaged in a device containing several compartments comprising:
- a first compartment containing a composition comprising one or more polymers containing silicone units and bearing alkoxy-(aminomethyl)-silyl functional groups, preferably of formula (I), at least one organic solvent, and less than 5% by weight of water relative to the total weight of the composition;
- a second compartment containing a composition comprising one or more catalysts as defined previously.

The device according to the invention may be intended for a one-stage or a two-stage application.

In the case of a one-stage application, the compositions of the first and second compartments may be dispensed simultaneously at the time of application.

Finally, the present invention relates to the use of a composition comprising one or more polymers containing silicone units and bearing alkoxy-(aminomethyl)-silyl functional groups, preferably of formula (I), at least one organic solvent, and less than 5% by weight of water relative to the total weight of the composition, for treating the hair, and in particular for shaping the hair.

The invention is illustrated in more detail in the following examples, which are provided by way of illustration and without implied limitation of the invention.

### Examples

The following compositions are prepared for performing the process of the invention, the contents being expressed on a weight basis relative to the total weight of the composition.

| **Examples** | **1** | **2** | **3** | **4** | **5** | **6** | **7** | **8** | **9** | **10** |
|---|---|---|---|---|---|---|---|---|---|---|
| Polymer (Iaa) | 5 | | | | | | | | | |
| Polymer (Iba) | | 5 | | | | | | | | |
| Polymer (Ica) | | | 5 | 5 | 10 | 10 | 32 | | | |
| Polymer (Ida) | | | | | | | | 5 | | |
| Polymer (Idb) | | | | | | | | | 5 | |
| Polymer (Idc) | | | | | | | | | | 5 |
| Isopropanol | | | 95 | | 90 | | 68 | 95 | | |
| Cyclopentadecamethylsilox ane | 95 | 95 | | 95 | | 90 | | | 95 | 95 |

| **Examples** | **11** | **12** | **13** | **14** | **15** | **16** | **17** | **18** | **19** | **20** |
|---|---|---|---|---|---|---|---|---|---|---|
| Polymer (Iaa) | 5 | | | | | | | | | |
| Polymer (Iba) | | 5 | | | | | | | | |
| Polymer (Ica) | | | 5 | | | | | | | |
| Polymer (Icb) | | | | 5 | | | | | | |
| Polymer (Icc) | | | | | 5 | | | | | |
| Polymer (Icd) | | | | | | 5 | | | | |
| Polymer (Ida) | | | | | | | 5 | | | |
| Polymer (Idb) | | | | | | | | 5 | | |
| Polymer (Idc) | | | | | | | | | 5 | |
| Polymer (Idd) | | | | | | | | | | 5 |
| Isododecane | | | 95 | 95 | 95 | 95 | 95 | 95 | 95 | 95 |
| Undecane/tridecane | 95 | 95 | | | | | | | | |

| **Examples** | **21** | **22** | **23** | **24** | **25** | **26** |
|---|---|---|---|---|---|---|
| Polymer (Ica) | 5 | 5 | 5 | 5 | 5 | 5 |
| 0.5N HCl as an aqueous solution | 1 | | | | | |
| 0.5N NaOH as an aqueous solution | | 1 | | | | |
| Citric acid at 8.7% in ethanol | | | 1 | | | |
| Citric acid at 8.7% as an aqueous solution | | | | 1 | | |
| Tartaric acid at 6.8% in ethanol | | | | | 1 | |
| Tartaric acid at 6.8% as an aqueous solution | | | | | | 1 |
| Isopropanol | 94 | 94 | 94 | 94 | 94 | 94 |

| **Examples** | **27** | **28** | **29** | **30** | **31** | **32** |
|---|---|---|---|---|---|---|
| Polymer (Ica) | 5 | 5 | 5 | 5 | 5 | 5 |
| APTES at 10% in ethanol | 1 | | | 1 | | |
| APTES at 10% in water | | | | | 1 | |
| APTES at 10% in water, lactic acid qs pH 10 | | | | | | 1 |
| Citric acid at 8.7% in ethanol | | 1 | | | | |
| Tartaric acid at 6.8% in ethanol | | | 1 | | | |
| Isododecane | 94 | 94 | 94 | 40 | 40 | 40 |
| Ethanol | | | | 54 | 54 | 54 |

Examples of compositions in the form of an aerosol system

| **Examples** | **33** | **34** | **35** | **36** | **37** | **38** | **39** |
|---|---|---|---|---|---|---|---|
| Polymer (Ica) | 4.4 | 4.4 | 4.4 | 4.4 | 4.4 | 4.4 | 17.6 |
| 0.1N HCl as an aqueous solution | 1 | | | | | | |
| Tartaric acid at 6.8% in ethanol | | 1 | | | | | |
| Tartaric acid at 6.8% as an aqueous solution | | | 1 | | | | |
| APTES* 10% in ethanol | | | | 1 | | | |
| APTES* 10% as an aqueous solution | | | | | 1 | | |
| Isopropanol | 49.6 | 49.6 | 49.6 | 49.6 | 49.6 | 50.6 | 37.4 |
| Dimethyl ether | 45 | 45 | 45 | 45 | 45 | 45 | 45 |

| | | | | | | | |
|---|---|---|---|---|---|---|---|
| *APTES: aminopropyltriethoxysilane | | | | | | | |

| **Examples** | **40** | **41** | **42** | **43** | **44** |
|---|---|---|---|---|---|
| Polymer (Ica) | 4.4 | 4.4 | 4.4 | 4.4 | 17.6 |
| 0.5N HCl as an aqueous solution | | | 1 | | |
| APTES* 10% as an aqueous solution | | | | 0.5 | |
| Isododecane | 50.6 | 22 | 49.6 | 49.1 | 37.4 |
| Ethanol | | 28.6 | | 0.5 | |
| Dimethyl ether | 45 | 45 | 45 | 45 | 45 |

| **Examples** | **45** | **46** | **47** | **48** | **49** | **50** | **51** |
|---|---|---|---|---|---|---|---|
| Polymer (Ice) | 5 | | | | 5 | 5 | 5 |
| Polymer (Icf) | | 5 | | | | | |
| Polymer (Icg) | | | 5 | | | | |
| Polymer (Ich) | | | | 5 | | | |
| APTES* 10% as an aqueous solution | | | | | 1 | | |
| APTES* 10% as a solution in ethanol | | | | | | 1 | |
| Isododecane | | 95 | 95 | 95 | | | 95 |
| Isopropanol | 95 | | | | 94 | 94 | |

Various application modes are envisaged:

### 1) One-stage application with a heating tool:

The compositions according to Examples 1 to 3, 13, 21 to 26, 27 to 32 and 45 to 51 were applied to locks of straight chestnut-brown Caucasian hair, with a bath ratio of 0.5.

The lock is predried and is then rolled up on a hot curling iron, at a temperature above 100°C, for 30 seconds, without adhesion to or degradation on the iron.

To evaluate the hold over time, the locks are suspended vertically over a paper and their relaxation is evaluated over time by marking the end of the lock on the paper.

To evaluate the resistance to moisture, the locks are placed in a chamber at controlled humidity (70%) for 24 hours.

The locks obtained with the compositions according to Examples 1 to 3, 13, 21 to 26 and 27 to 32 have a cohesive curl, i.e. the hairs are stuck together in the curl.

The locks treated with the compositions according to Examples 1 to 3, 13, 21 to 26 and 27 to 32 have very good hold over time and resistance to moisture.

The use of a hairdryer to perform blow-drying, and the use of flat heating tongs, instead of a curling iron, makes it possible to obtain another type of styling, characterized by a soft feel, very good discipline, namely with a parallel alignment of the hairs, and which withstands moisture.

Compositions 3, 8, 13, 14, 17, 19, 48 and 51 were applied to 2.7 g curly locks, washed beforehand and manually dried but still humid, with a bath ratio of 0.4. Blow-drying was then performed until the hair appeared dried. The hairs of the locks thus treated are straight and aligned, and the effect is preserved after 24 hours of exposure to a humidity of 70%.

Compositions 13, 14, 48 and 51 were applied to 2.7 g curly locks, washed beforehand and manually dried but still humid, with a bath ratio of 0.4. Blow-drying was then performed until the hair appeared dry, followed by ten treatments with flat tongs at 210°C. The hairs of the locks thus treated are straight and aligned, and the effect is preserved after 24 hours of exposure to a humidity of 70%.

### 2) Application of styling product without heating

The application may also be performed in one stage on short hair, without heating, to obtain a styling effect.

### 3) Application as an aerosol

The composition according to Examples 33 to 38 and 40 to 43 was applied to a lock of natural straight hair 27 cm long, weighing 5.4 g, with a separation of 15 cm, for 3 seconds. Good fixing combined with a soft cosmetic feel, which is not tacky after drying, easy to remove by brushing and not having any residues, was obtained.

The composition according to Example 39 or 44 was applied to medium-length hair, at 15 cm, for 6 to 8 seconds on each side. Good fixing combined with a soft cosmetic feel, which is not tacky, easy to disentangle by simple brushing and not having any residues, was thus obtained.

Compositions according to examples 3, 13 and 52 to 58 below have been applied to locks of straight natural wet hair, with a bath ratio of 0.5.

Each lock has been predried and then rolled up on a hot curling iron, at a temperature above 100 C, for 30 seconds.

To evaluate the hold of the curl over time, the locks have been suspended vertically over a paper and their relaxation has been evaluated over time by marking the end of the lock on the paper. Then, the locks have been placed vertically in a chamber at controlled humidity (80%) for 24 hours. The locks have then been suspended vertically over a paper again and their relaxation evaluated by marking the end of the lock on the paper.

To evaluate the resistance to moisture of the curl, the difference between the length of the lock before its introduction in the chamber and after 24h in the chamber has been measured and the shape of the curls observed.

The results are indicated in the table below :

| | **3** | **52** | **53** | **54** | **55** | **56** | **57** | **58** | **13** |
|---|---|---|---|---|---|---|---|---|---|
| Polymer (Ica) | 5 | 5 | 5 | 5 | 5 | - | 5 | - | 5 |
| Isododecane | - | - | - | - | - | - | - | 100 | qsp |
| Isopropanol | qsp | qsp | qsp | qsp | qsp | 100 | - | - | - |
| Water | - | 1 | 2 | 5 | 10 | | qsp | - | - |
| Resistance to moisture of the curl | ○ | ○ | ○ | ▲ | ● | ● | ● | ● | ○ |

| | | | | | | | | | |
|---|---|---|---|---|---|---|---|---|---|
| ○ Good (Few or no elongation of the lock and few or no modification of the shape of the curls) ▲ insufficient (important elongation of the lock and important modification of the shape of the curls) ● Bad (very important elongation of the lock and important modification of the shape of the curls) | | | | | | | | | |

## Claims

1. Hair treatment process using a composition comprising:
- at least one organic solvent,
- less than 2% by weight of water relative to the total weight of the composition,
- one or more polymers containing silicone units and bearing alkoxy-(aminomethyl)-silyl functional groups of formula (I) below: 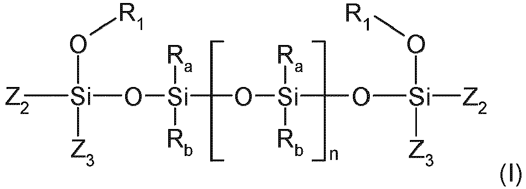 in which:
- Z₂ represents a group -CH₂-NR₃R₄;
- Z₃ represents a group OR₅ or R₆;
- R₃, which may be identical or different, represent a hydrogen atom or a group R₇;
- R₁, R₅, R₆ and R₇, which may be identical or different, represent a C₁-C₆ alkyl group and R₄ represents a C₁-C₆ alkyl group or a C₅-C₆ cycloalkyl group;
R₃ and R₄ possibly forming, with the nitrogen atom that bears them, a 5- to 8-membered heterocycle comprising from 1 to 3 heteroatoms,
Rₐ and R_{b}, which may be identical or different, represent a C₁-C₂ alkyl group,
n ranges from 5 to 1000
in which SiRₐR_{b}-[OSiRₐR_{b}]n- of formula (I) is a unit derived from a linear silicone with a weight-average molecular mass (Mw) ranging from 400 to 25 000,
the polymer(s) containing silicone units and bearing alkoxy-(aminomethyl)-silyl functional groups, of formula (I), represent from 0.5% to 30% by weight relative to the total weight of the composition.

2. Process according to Claim 1, in which the C₁-C₆ alkyl groups are methyl or ethyl groups.

3. Process according to either of Claims 1 and 2, in which in formula(I) R₄ represents a C₆ cycloalkyl group such as cyclohexyl.

4. Process according to any one of Claims 1 to 3, in which in formula (I) n ranges from 8 to 400.

5. Process according to any one of the preceding claims, in which the polymer(s) containing silicone units and bearing alkoxy-(aminomethyl)-silyl functional groups, of formula (I), represent from 1% to 10% by weight relative to the total weight of the composition.

6. Process according to any one of the preceding claims, in which the organic solvent is chosen from alcohols, alkanes, esters and silicones, and mixtures thereof, preferably from ethanol, propanol, isopropanol, glycerol, undecane, tridecane, isododecane, isopropyl myristate, ethyl adipate, ethyl acetate, linear low-molecular-weight silicones or cyclic silicones such as cyclopentasiloxane, and also mixtures thereof, preferably from ethanol, isopropanol and glycerol, and mixtures thereof.

7. Process according to any one of the preceding claims, **characterized in that** the solvent(s) represent from 10% to 99.8%, preferably from 30% to 98% by weight and better still from 35% to 95% by weight relative to the total weight of the composition.

8. Process according to any one of the preceding claims, **characterized in that** it uses one or more catalysts, the catalyst(s) preferably being chosen from organic or mineral basic compounds, especially ammonia or sodium hydroxide, organic or mineral acids, especially hydrochloric acid, oleic acid or lactic acid, and mixtures thereof.

9. Process according to Claim 8, **characterized in that** the catalyst(s) are present in the composition comprising the polymer(s) containing silicone units and bearing alkoxy-(aminomethyl)-silyl functional groups.

10. Process according to either of Claims 8 and 9, **characterized in that** the catalyst(s) are present in a content ranging from 0.0001% to 10% by weight, preferably from 0.001% to 5% by weight and better still from 0.01% to 2% by weight relative to the total weight of the composition containing them.

11. Process according to the preceding claim, which also comprises a step of heating the hair using a heating tool chosen from a straightening iron, a curling iron, a crimping iron, a waving iron, a hood and a hairdryer.

12. Use of a composition comprising,
- at least one organic solvent,
- less than 2% by weight of water relative to the total weight of the composition,
- one or more polymers containing silicone units and bearing alkoxy-(aminomethyl)-silyl functional groups of formula (I) below: 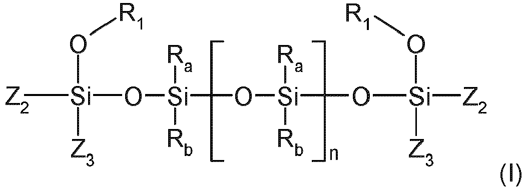 in which:
- Z₂ represents a group -CH₂-NR₃R₄;
- Z₃ represents a group OR₅ or R₆;
- R₃, which may be identical or different, represent a hydrogen atom or a group R₇;
- R₁, R₅, R₆ and R₇, which may be identical or different, represent a C₁-C₆ alkyl group and R₄ represents a C₁-C₆ alkyl group or a C₅-C₆ cycloalkyl group;
R₃ and R₄ possibly forming, with the nitrogen atom that bears them, a 5- to 8-membered heterocycle comprising from 1 to 3 heteroatoms,
Rₐ and R_{b}, which may be identical or different, represent a C₁-C₂ alkyl group,
n ranges from 5 to 1000
in which SiRₐR_{b}-[OSiRₐR_{b}]n- of formula (I) is a unit derived from a linear silicone with a weight-average molecular mass (Mw) ranging from 400 to 25 000,
the polymer(s) containing silicone units and bearing alkoxy-(aminomethyl)-silyl functional groups, of formula (I), represent from 0.5% to 30% by weight relative to the total weight of the composition,
for treating the hair, and in particular for shaping the hair.

## Patentansprüche

1. Haarbehandlungsverfahren unter Verwendung einer Zusammensetzung, umfassend:
- mindestens ein organisches Lösungsmittel,
- weniger als 2 Gew.-% Wasser, bezogen auf das Gesamtgewicht der Zusammensetzung,
- ein oder mehrere Polymere, die Silikoneinheiten enthalten und alkoxy(aminomethyl)silylfunktionelle Gruppen der nachstehenden Formel (I) tragen: 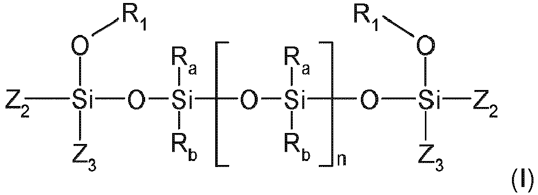 wobei:
- Z₂ für eine Gruppe -CH₂-NR₃R₄ steht;
- Z₃ für eine Gruppe OR₅ oder R₆ steht;
- R₃, das gleich oder verschieden sein kann, für ein Wasserstoffatom oder eine Gruppe R₇ steht;
- R₁, R₅, R₆ und R₇, die gleich oder verschieden sein können, für eine C₁-C₆-Alkylgruppe stehen und R₄ für eine C₁-C₆-Alkylgruppe oder eine C₅-C₆-Cycloalkylgruppe steht;
wobei R₃ und R₄ gegebenenfalls mit dem Stickstoffatom, das sie trägt, einen 5- bis 8-gliedrigen Heterocyclus mit 1 bis 3 Heteroatomen bilden,
Rₐ und R_{b}, die gleich oder verschieden sein können, für eine C₁-C₂-Alkylgruppe stehen,
n im Bereich von 5 bis 1000 liegt,
wobei SiRₐR_{b}-[OSiRₐR_{b}]n- der Formel (I) eine Einheit ist, die sich von einem linearen Silikon mit einer gewichtsmittleren Molekularmasse (Mw) von 400 bis 25.000 ableitet,
wobei das Polymer bzw. die Polymere, das bzw. die Silikoneinheiten enthält bzw. enthalten und alkoxy(aminomethyl)silylfunktionelle Gruppen der Formel (I) trägt bzw. tragen, 0,5 bis 30 Gew.-%, bezogen auf das Gesamtgewicht der Zusammensetzung, ausmacht bzw. ausmachen.

2. Verfahren nach Anspruch 1, wobei es sich bei den C₁-C₆-Alkylgruppen um Methyl- oder Ethylgruppen handelt.

3. Verfahren nach Anspruch 1 oder 2, wobei in Formel (I) R₄ für eine C₆-Cycloalkylgruppe wie Cyclohexyl steht.

4. Verfahren nach einem der Ansprüche 1 bis 3, wobei in Formel (I) n im Bereich von 8 bis 400 liegt.

5. Verfahren nach einem der vorhergehenden Ansprüche, wobei das Polymer bzw. die Polymere, das bzw. die Silikoneinheiten enthält bzw. enthalten und alkoxy(aminomethyl)silylfunktionelle Gruppen der Formel (I) trägt bzw. tragen, 1 bis 10 Gew.-%, bezogen auf das Gesamtgewicht der Zusammensetzung, ausmacht bzw. ausmachen.

6. Verfahren nach einem der vorhergehenden Ansprüche, wobei das organische Lösungsmittel aus Alkoholen, Alkanen, Estern und Silikonen und Mischungen davon, vorzugsweise aus Ethanol, Propanol, Isopropanol, Glycerin, Undecan, Tridecan, Isododecan, Isopropylmyristat, Ethyladipat, Ethylacetat, linearen niedermolekularen Silikonen oder cyclischen Silikonen wie Cyclopentasiloxan sowie Mischungen davon, vorzugsweise aus Ethanol, Isopropanol und Glycerin und Mischungen davon, ausgewählt wird.

7. Verfahren nach einem der vorhergehenden Ansprüche, **dadurch gekennzeichnet, dass** das Lösungsmittel bzw. die Lösungsmittel 10 bis 99,8 Gew.-%, vorzugsweise 30 bis 98 Gew.-% und noch besser 35 bis 95 Gew.-%, bezogen auf das Gesamtgewicht der Zusammensetzung, ausmacht bzw. ausmachen.

8. Verfahren nach einem der vorhergehenden Ansprüche, **dadurch gekennzeichnet, dass** dabei ein oder mehrere Katalysatoren verwendet werden, wobei der Katalysator bzw. die Katalysatoren vorzugsweise aus organischen oder mineralischen basischen Verbindungen, insbesondere Ammoniak oder Natriumhydroxid, organischen Säuren oder Mineralsäuren, insbesondere Salzsäure, Ölsäure oder Milchsäure, und Mischungen davon ausgewählt wird bzw. werden.

9. Verfahren nach Anspruch 8, **dadurch gekennzeichnet, dass** der Katalysator bzw. die Katalysatoren in der Zusammensetzung, die das Polymer bzw. die Polymere, das bzw. die Silikoneinheiten enthält bzw. enthalten und alkoxy(aminomethyl)silylfunktionelle Gruppen trägt bzw. tragen, umfasst, vorliegt bzw. vorliegen.

10. Verfahren nach Anspruch 8 oder 9, **dadurch gekennzeichnet, dass** der Katalysator bzw. die Katalysatoren in einem Gehalt im Bereich von 0,0001 bis 10 Gew.-%, vorzugsweise von 0,001 bis 5 Gew.-% und noch besser von 0,01 bis 2 Gew.-%, bezogen auf das Gesamtgewicht der Zusammensetzung, die sie enthält, vorliegen.

11. Verfahren nach dem vorhergehenden Anspruch, das auch einen Schritt des Erhitzens des Haars unter Verwendung eines Heizwerkzeugs, das aus einem Glätteisen, einem Lockenstab, einem Kräuseleisen, einem Welleisen, einer Trockenhaube und einem Haartrockner ausgewählt wird, umfasst.

12. Verwendung einer Zusammensetzung, umfassend
- mindestens ein organisches Lösungsmittel,
- weniger als 2 Gew.-% Wasser, bezogen auf das Gesamtgewicht der Zusammensetzung,
- ein oder mehrere Polymere, die Silikoneinheiten enthalten und alkoxy(aminomethyl)silylfunktionelle Gruppen der nachstehenden Formel (I) tragen: 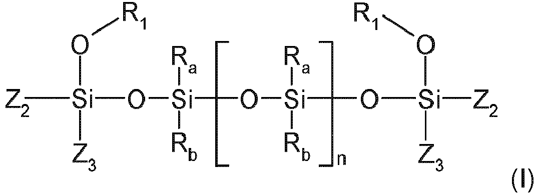 wobei:
- Z₂ für eine Gruppe -CH₂-NR₃R₄ steht;
- Z₃ für eine Gruppe OR₅ oder R₆ steht;
- R₃, das gleich oder verschieden sein kann, für ein Wasserstoffatom oder eine Gruppe R₇ steht;
- R₁, R₅, R₆ und R₇, die gleich oder verschieden sein können, für eine C₁-C₆-Alkylgruppe stehen und R₄ für eine C₁-C₆-Alkylgruppe oder eine C₅-C₆-Cycloalkylgruppe steht;
wobei R₃ und R₄ gegebenenfalls mit dem Stickstoffatom, dass sie trägt, einen 5- bis 8-gliedrigen Heterocyclus mit 1 bis 3 Heteroatomen bilden,
Rₐ und R_{b}, die gleich oder verschieden sein können, für eine C₁-C₂-Alkylgruppe stehen,
n im Bereich von 5 bis 1000 liegt,
wobei SiRₐR_{b}-[OSiRₐR_{b}]n- der Formel (I) eine Einheit ist, die sich von einem linearen Silikon mit einer gewichtsmittleren Molekularmasse (Mw) von 400 bis 25.000 ableitet,
wobei das Polymer bzw. die Polymere, das bzw. die Silikoneinheiten enthält bzw. enthalten und alkoxy(aminomethyl)silylfunktionelle Gruppen der Formel (I) trägt bzw. tragen, 0,5 bis 30 Gew.-%, bezogen auf das Gesamtgewicht der Zusammensetzung, ausmacht bzw. ausmachen,
zur Behandlung des Haars, insbesondere zum Formen des Haars.

## Revendications

1. Procédé de traitement des cheveux utilisant une composition comprenant
- au moins un solvant organique,
- moins de 2 % en poids d'eau par rapport au poids total de la composition,
- un ou plusieurs polymères contenant des motifs de silicone et portant des groupes fonctionnels alcoxy-(aminométhyl)-silyle de formule (I) ci-dessous : 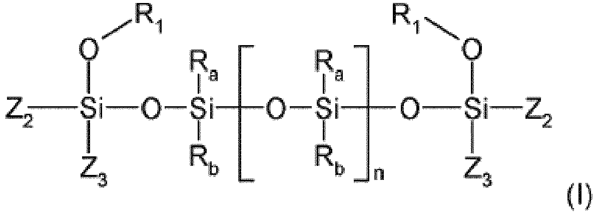 dans laquelle :
- Z₂ représente un groupe -CH₂-NR₃R₄ ;
- Z₃ représente un groupe OR₅ ou R₆ ;
- R₃, qui peuvent être identiques ou différents, représentent un atome d'hydrogène ou un groupe R₇ ;
- R₁, R₅, R₆, R₇, qui peuvent être identiques ou différents, représentent un groupe alkyle en C₁-C₆, et R₄ représente un groupe alkyle en C₁-C₆ ou un groupe cycloalkyle en C₅-C₆ ;
R₃ et R₄ pouvant former avec l'atome d'azote qui les porte un hétérocycle de 5 à 8 chaînons et comprenant 1 à 3 hétéroatomes,
Rₐ et R_{b}, qui peuvent être identiques ou différents, représentent un groupe alkyle en C₁-C₂, n se situe dans la plage de 5 à 1 000
dans laquelle SiRₐR_{b}-[OSiRₐR_{b})]n- de la formule (I) est un motif issu d'une silicone linéaire dotée d'une masse moléculaire moyenne en poids (Mw) dans la plage de 400 à 25 000,
le ou les polymères contenant des motifs de silicone et portant des groupes fonctionnels alcoxy-(aminométhyl)-silyle, de formule (I), représentent de 0,5 % à 30 % en poids par rapport au poids total de la composition.

2. Procédé selon la revendication 1, dans lequel les groupes alkyle en C₁-C₆ sont des groupes méthyle ou éthyle.

3. Procédé selon l'une ou l'autre des revendications 1 et 2, dans lequel dans la formule (I), R₄ représente un groupe cycloalkyle en C₆ tel que cyclohexyle.

4. Procédé selon l'une quelconque des revendications 1 à 3, dans lequel dans la formule (I), n se situe dans la plage de 8 à 400.

5. Procédé selon l'une quelconque des revendications précédentes, dans lequel le ou les polymères contenant des motifs de silicone et portant des groupes fonctionnels alcoxy-(aminométhyl)-silyle, de formule (I), représentent de 1 % à 10 % en poids par rapport au poids total de la composition.

6. Procédé selon l'une quelconque des revendications précédentes, dans lequel le solvant organique est choisi parmi des alcools, des alcanes, des esters et des silicones, et des mélanges correspondants, préférablement parmi l'éthanol, le propanol, l'isopropanol, le glycérol, l'undécane, le tridécane, l'isododécane, le myristate d'isopropyle, l'adipate d'éthyle, l'acétate d'éthyle, des silicones linéaires à bas poids moléculaire ou des silicones cycliques telles que le cyclopentasiloxane, et également des mélanges correspondants, préférablement parmi l'éthanol, l'isopropanol et le glycérol et des mélanges correspondants.

7. Procédé selon l'une quelconque des revendications précédentes, **caractérisé en ce que** le ou les solvants représentent de 10 % à 99,8 %, préférablement de 30 % à 98 % en poids et encore mieux de 35 % à 95 % en poids par rapport au poids total de la composition.

8. Procédé selon l'une quelconque des revendications précédentes, **caractérisé en ce qu'**il utilise un ou plusieurs catalyseurs, le ou les catalyseurs étant préférablement choisis parmi des composés basiques organiques ou minéraux, notamment l'ammoniaque ou l'hydroxyde de sodium, des acides organiques ou minéraux, notamment l'acide chlorhydrique, l'acide oléique ou l'acide lactique, et des mélanges correspondants.

9. Procédé selon la revendication 8, **caractérisé en ce que** le ou les catalyseurs sont présents dans la composition comprenant le ou les polymères contenant des motifs de silicone et portant des groupes fonctionnels alcoxy-(aminométhyl)-silyle.

10. Procédé selon l'une ou l'autre des revendications 8 et 9, **caractérisé en ce que** le ou les catalyseurs sont présents en une teneur dans la plage de 0,0001 % à 10 % en poids, préférablement de 0,001 à 5 % en poids et encore mieux de 0,01 à 2 % en poids du poids total de la composition qui les contient.

11. Procédé selon la revendication précédente, qui comprend également une étape de chauffage des cheveux en utilisant un outil de chauffage choisi parmi un fer à lisser, un fer à friser, un fer à gaufrer, un fer à onduler, une hotte et un séchoir à cheveux.

12. Utilisation d'une composition comprenant,
- au moins un solvant organique,
- moins de 2 % en poids d'eau par rapport au poids total de la composition,
- un ou plusieurs polymères contenant des motifs de silicone et portant des groupes fonctionnels alcoxy-(aminométhyl)-silyle de formule (I) ci-dessous : 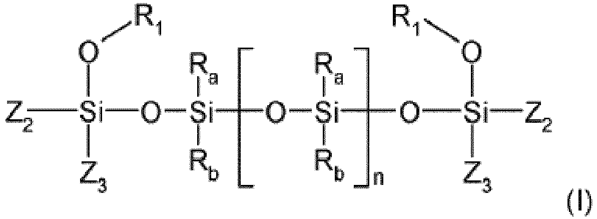 dans laquelle :
- Z₂ représente un groupe -CH₂-NR₃R₄ ;
- Z₃ représente un groupe OR₅ ou R₆ ;
- R₃, identiques ou différents, représentent un atome d'hydrogène ou un groupe R₇ ;
- R₁, R₅, R₆, R₇, qui peuvent être identiques ou différents, représentent un groupe alkyle en C₁-C₆, et R₄ représente un groupe alkyle en C₁-C₆ ou un groupe cycloalkyle en C₅-C₆ ;
R₃ et R₄ pouvant former avec l'atome d'azote qui les porte un hétérocycle de 5 à 8 chaînons et comprenant 1 à 3 hétéroatomes,
Rₐ et R_{b}, qui peuvent être identiques ou différents, représentent un groupe alkyle en C₁-C₂,
n se situe dans la plage de 5 à 1 000
dans laquelle SiRₐR_{b}-[OSiRₐR_{b})]n- de la formule (I) est un motif issu d'une silicone linéaire dotée d'une masse moléculaire moyenne en poids (Mw) dans la plage de 400 à 25 000
le ou les polymères contenant des motifs de silicone et portant des groupes fonctionnels alcoxy-(aminométhyl)-silyle, de formule (I), représentent de 0,5 % à 30 % en poids par rapport au poids total de la composition,
pour un traitement des cheveux et en particulier pour une mise en forme des cheveux.
